(19) Europäisches Patentamt
European Patent Office
Office européen des brevets

(11) Numéro de publication: **0 169 785 B1**

# (12) FASCICULE DE BREVET EUROPEEN

(45) Date de publication du fascicule du brevet: **18.10.89**

(51) Int. Cl.⁴: **A 61 K 31/225**

(21) Numéro de dépôt: **85401495.8**

(22) Date de dépôt: **19.07.85**

(54) **Utilisation du triacétoxy-1,8,9 anthracène comme ingredient actif dans la préparation d'un médicament anti-tumoral.**

(30) Priorité. **20.07.84 FR 8411519**

(43) Date de publication de la demande:
**29.01.86 Bulletin 86/5**

(45) Mention de la délivrance du brevet:
**18.10.89 Bulletin 89/42**

(84) Etats contractants désignés:
**BE CH DE FR GB IT LI NL SE**

(56) Documents cités:
**ARZNEIMITTELFORSCHUNG, vol. 20, no. 1, janvier 1970, pages 137-139, Aulendorf, DE; M. WHITEFIELD: "The treatment of psoriasis with triacetoxyanthracene"**
**CHEMICAL ABSTRACTS, vol. 81, 1974, page 20, no. 20859n, Columbus, Ohio, US; W. RAAB et al.: "Glucose 6-phosphate dehydrogenase and topical antipsoriatics. Comparison of anthralin, anthralin monoacetate, anthralin diacetate, anthralin triacetate, and 6-hydroxy-2-oxobenzoxathiole", & ARCH. DERMATOL. FORSCH. 1974, 249(2), 179-89**
**CHEMICAL ABSTRACTS, vol. 74, 1971, page 241, no. 40976g, Columbus, Ohio, US; D.T. WALZ et al.: "Arthritis and psoriasis: effects of antipsoriatic agents in the adjuvant-induced arthritic rat", & PROC. SOC. EXP. BIOL. MED. 1970, 135(3), 760-2**

(73) Titulaire: **CENTRE INTERNATIONAL DE RECHERCHES DERMATOLOGIQUES C.I.R.D. Groupement d'Intérêt Economique dit:, Sophia Antipolis, F-06560 Valbonne (FR)**

(72) Inventeur: **Shroot, Braham Villa 35 Hameaux de Val-Bosquet, Chemin de Val-Bosquet, F-06600 Antibes (FR)**
Inventeur: **Lang, Gérard, 26 ter, avenue Jean-Jaurès, F-93800 Epinay-Sur-Seine (FR)**
Inventeur: **Maignan, Jean, 8, rue Halévy, F-93290 Tremblay Les Gonesse (FR)**

(74) Mandataire: **Nony, Michel et al, Cabinet Nony 29, rue Cambacérès, F-75008 Paris (FR)**

**EP 0 169 785 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet l'utilisation du triacétoxy-1,8,9 anthracène comme ingrédient actif dans la préparation d'un médicament anti-tumoral.

Le triacétoxy-1,8,9 anthracène a été préparé et décrit par O. E. Schultz et H. H. Schultze-Mosgau, Archive der Pharmazie, 298, 313-320 (1965).

Il répond à la formule I:

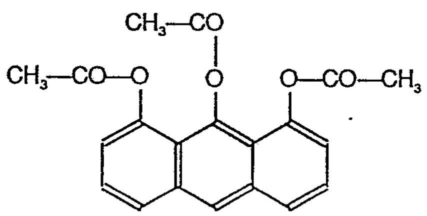

(I)

Ce composé se présente sous la forme de cristaux incolores; P.F. = 217-218°C. Il est soluble notamment dans les alcools et l'acétone.

D'autre part, l'activité anti-psoriasis, en application par voie topique, de ce produit a été décrite par M. Whitefield, Arzneim.-Forsch. 20, 137 (1970).

On a maintenant découvert que le composé de formule I possède une activité cytostatique vis-à-vis des cellules tumorales qui permet de préconiser son utilisation en chimiothérapie anticancéreuse.

La présente invention a pour objet l'utilisation du triacétoxy 1,8,9 anthracène comme ingrédient actif dans la préparation d'un médicament anti-tumoral, en association avec un véhicule approprié pour l'administration par voie parentérale ou orale.

Dans le médicament anti-tumoral de l'invention, la concentration de l'ingrédient actif varie généralement de 0,01 à 70% en poids, en fonction de la forme pharmaceutique ou de la voie d'administration choisie.

Ce médicament peut se présenter notamment sous la forme de solutions ou de suspensions injectables ou sous la forme de suspensions buvables, de comprimés, de poudres, de gélules, de capsules, de granulés ou de sirops.

Ces formes pharmaceutiques sont préparées selon les méthodes usuelles.

Le composé de formule I peut également être administré après incorporation dans des liposomes qui sont préparés par exemple à partir de lécithine d'œuf, de cholestérol et de phosphate de dicétyle.

La posologie varie notamment en fonction de l'affection traitée et de la forme d'administration.

Pour la voie parentérale ou orale, on administre généralement de 0,005 à 5 g d'ingrédient actif par jour chez l'adulte, en une ou plusieurs fois.

Le médicament anti-tumoral obtenu selon l'invention est en particulier un médicament conditionné et préparé industriellement, tel que défini ci-dessus, caractérisé par le fait qu'il comprend la composition active, dans un emballage approprié, avec une notice d'emploi mentionnant le mode d'administration préconisé pour son utilisation en chimiothérapie anti-cancéreuse.

L'invention a également pour objet l'utilisation du composé de formule I dans la préparation industrielle d'un médicament conditionné tel que défini précédemment.

Les exemples suivants illustrent l'invention:

Exemple 1

Comprimé à avaler ou à croquer

On a préparé des comprimés de 0,8 g ayant la composition suivante:

| | |
|---|---|
| Composé I | 0,500 g |
| Amidon prégélatinisé | 0,100 g |
| Cellulose microcristalline | 0,115 g |
| Lactose | 0,075 g |
| Stéarate de magnésium | 0,010 g |

Exemple 2

Poudre en sachets

| | |
|---|---|
| Composé I | 0,300 g |
| Gélatine | 0,200 g |
| Saccharose | 0,500 g |
| Agent d'aromatisation | q.s. |

Exemple 3

Suspension injectable par voie intramusculaire en ampoules

| | |
|---|---|
| Composé I soniqué | 0,005 g |
| Tampon | pH 6 |
| Chlorure de sodium | 0,072 g |
| Eau pour préparation injectable q.s.p. | 8 ml |

Exemple 4

Etude de l'activité anti-tumorale vis-à-vis des cellules leucémiques lymphocytiques P 388.

Le principe du test est le suivant: On implante chez la souris, au jour zéro, une tumeur, par injection intrapéritonéale de $10^6$ cellules tumorales dans 0,1 ml de fluide ascitique dilué. Le traitement consiste à administrer quotidiennement le produit étudié, par voie intrapéritonéale pendant les 5 jours suivant l'implantation de la tumeur.

Les résultats du test sont résumés dans le tableau I suivant:

Tableau I

| Posologie mg/kg | Activité | | |
|---|---|---|---|
| | Temps médian de survie (en jours) | | $\frac{T}{C} \times 100$ |
| | Animaux testés T | Animaux témoins C | |
| 3,12 | 13 | 9,9 | 131 |
| 1,56 | 13,3 | 9,9 | 134 |
| 0,79 | 12,3 | 9,9 | 124 |

On considère que le produit est actif selon ce test, lorsque le rapport

$$\frac{T}{C} \times 100$$

est supérieur à 120.

Cette étude montre que le composé de formule I est actif à la dose de 0,8 mg/kg environ et que la dose optimale est voisine de 1,56 mg/kg.

Exemple 5

Etude de l'activité anti-tumorale vis-à-vis des cellules de mélanocarcinome B 16.

Le principe du test est le suivant: On a implanté, chez la souris, par injection intrapéritonéale au jour 0, 0,5 ml d'un homogénéisat dilué (1/10) des cellules tumorales.

Le traitement consiste à administrer le produit étudié, quotidiennement, par voie intrapéritonéale, pendant les 9 jours suivant l'implantation de la tumeur.

Les résultats sont résumés dans le tableau II suivant:

Tableau II

| Posologie mg/kg | Activité | | |
|---|---|---|---|
| | Temps médian de survie (en jours) | | $\frac{T}{C} \times 100$ |
| | Animaux testés T | Animaux témoins C | |
| 3 | 20 | 15,4 | 129 |
| 1,5 | 23 | 15,4 | 149 |
| 0,75 | 19,7 | 15,4 | 127 |

On considère qu'un produit est actif, selon ce test, lorsque le rapport

$$\frac{T}{C} \times 100$$

est supérieur à 125.

Cette étude montre que le composé de formule I est actif à la dose de 0,75 mg/kg et que la dose optimale est voisine de 1,50 mg/kg.

## Revendications

1. Utilisation du triacétoxy-1,8,9 anthracène comme ingrédient actif en association avec un véhicule approprié à l'administration par voie parentérale ou orale dans la préparation d'un médicament anti-tumoral.

2. Utilisation selon la revendication 1, caractérisé par le fait que la concentration de l'ingrédient actif est de 0,01 à 70% en poids.

3. Utilisation selon l'une quelconque des revendications précédentes, caractérisée par le fait que le médicament se présente sous la forme de solutions ou suspensions injectables, de comprimés, de gélules, de capsules, de poudres, de suspensions buvables, de granulés ou de sirops.

## Patentansprüche

1. Verwendung von Triacetoxy-1,8,9-anthracen als Wirkstoff zusammen mit einem zur parenteralen oder oralen Verabreichung geeigneten Träger zur Herstellung eines Antitumorarzneimittels.

2. Verwendung nach Anspruch 1, dadurch gekennzeichnet, dass die Konzentration des Wirkstoffs 0,01 bis 70 Gew.-% beträgt.

3. Verwendung nach einem der vorhergehenden Ansprüche, dadurch gekennzeichnet, dass das Arzneimittel in Form von injizierbaren Lösungen oder Suspensionen, Tabletten, Gelatinekapseln, Kapseln, Pulver, trinkbaren Suspensionen, Granula oder als Sirup vorliegt.

## Claims

1. Use of 1,8,9-triacetoxyanthracene as active ingredient, in combination with an appropriate vehicle for parenteral or oral administration, in the preparation of an anti-tumour medicament.

2. Use according to claim 1, characterized by the fact that the concentration of the active ingredient is from 0.01 to 70% by weight.

3. Use according to either one of the preceding claims, characterized by the fact that the medicament is in the form of injectable solutions or suspensions, tablets, soft capsules, hard capsules, powders, drinkable suspensions, granules or syrups.